# EUROPEAN PATENT SPECIFICATION

(11) **EP 3 572 111 B1**
(45) Date of publication and mention of the grant of the patent: **28.06.2023**
(21) Application number: 18173703.2
(22) Date of filing: 22.05.2018
(51) Int. Cl.: A61M 16/04, A61M 25/02

(54) **MEDICAL LINE SECUREMENT DEVICE**
BEFESTIGUNGSVORRICHTUNG FÜR EINE MEDIZINISCHE LEITUNG
DISPOSITIF DE FIXATION DE TUBE MEDICAL

(43) Date of publication of application: 27.11.2019
(73) Proprietor: Novo Klinik-Service GmbH, 50127 Bergheim (DE)
(72) Inventor: Van der Vegt, Herman, 3512 LL Utrecht (NL); Teunissen, Wilfred, 3514 AD Utrecht (NL); Van Oudheusden, Freerk, 2215 RA Voorhout (NL)
(74) Representative: Kesselhut, Wolf

(56) References cited:
- EP-A1- 0 356 683
- WO-A1-2010/033109
- WO-A1-2012/064732
- WO-A2-2014/183060
- CN-A- 107 929 903
- US-A- 2 820 457
- US-A- 5 653 232

## Description

### TECHNICAL FIELD

The present disclosure relates to the field of fixing an intubation line or other line to a patient, in particular to the field of endotracheal tube fixation devices.

### BACKGROUND

Patients that are incapable of breathing may be ventilated via intubation, i.e. placement of an endotracheal tube into the throat of the patient. E.g. for proper ventilation of the patient and/or for proper control of the ventilation and/or for prevention of harm to the patient by the inserted foreign object the position of the tube must be reliably fixed to the patient, both in longitudinal direction of the tube and in radial direction thereto. Similar considerations apply for other lines introduced into a patient, e.g. feeding lines, sensor probes, catheters, drains, etc.

Medicinal lines may be in use for prolonged periods of care and patient-friendliness is therefore an important consideration. The line must be exchangeable for adaptation, for cleaning of the patient and/or the line, for adjustment of treatment, for administration of medicaments and/or food etc., and similar instances. User friendliness for care givers is therefore important as well.

Moreover, various types and/or sizes of tubes may be used for different treatments. Further, intubation must sometimes be applied under significant pressure and haste, e.g. in case of an accident and/or in case of short staffing.

The various operating conditions that may be encountered therefore have led to commercially available fixation device optimised for different uses.

An exemplary securement system for an endotracheal tube is disclosed in WO 2010/033109 A1: a securement system securing an endotracheal tube or other medical article in position upon a patient and arresting movement of the endotracheal tube. The securement system includes a support member and a retainer. The retainer defines a channel configured to receive the endotracheal tube. A head securement member attaches the support member to the head of the patient. The securement system can include a soft gel for placement against the patient's skin and a track for securing the endotracheal tube at a plurality of locations relative to the support member. The securement system can maintain an endotracheal tube in position upon a patient and allow access to the patient's oral cavity.

Herewith, further improvements to endotracheal tube fixation are provided.

It is noted that US 5,653,232 discloses an endotracheal tube positioner; US 2,820,457 discloses a positioning retainer for oro-tracheal tubes; CN 107 929 903 discloses an oral tube intubation fixing device; EP 0 356 683 discloses an attachment device, particularly for percutaneous conduits, such as catheters, drains, tubes and the like, on the skin of a patient; WO 2012/064732 discloses an endotracheal tube holder.

### SUMMARY

The present invention provides a fixation device for fixing a medical line to a patient as defined in claim 1. The medical line may be a wire, a tube or similar. In particular the medical line may comprise an endotracheal tube and/or a gastric tube and/or the line may be associated with a lanyngeal mask, e.g. as an integral whole. The fixation device comprises a support member supporting a retainer, the retainer defining an axis and being adapted to retain the medical line extending in axial direction. The retainer comprises an enclosure for radially retaining the line and a clamp. The clamp is configured to provide a crimping force around the axis for fixing the line in at least axial direction, preferably also radial direction, relative to the enclosure. Herein, terms like axial and radial relate to the axis.

The crimping force is a centripetal force in a net radial direction towards the axis, e.g. a summation of plural localised radial force contributions distributed over more than 180 degrees around the axis.

Thus, the fixation device can provide radial retainment and axial fixation, preferably also radial fixation. This facilitates initial placement of the line, possibly adjustment of the line to a desired position and/or orientation relative to the patient and/or to the enclosure, and fixation of the line by the clamp. The device can clamp the line without localised or one-sided pinching force, e.g. preventing risks of closing or damaging a tube e.g. by squeezing and/or buckling.

The clamp may be configured to provide a substantially axisymmetric clamping force distribution. This may center the line and facilitate positioning it.

The clamp comprises a deformable member which may be tightenable in circumferential direction around the axis. The deformable member comprises one of a flexible strap, a belt and a chain. This may facilitate conforming the clamp to a shape of the line, e.g. in case the line comprise two line parts with different sizes adjacent each other such as a multibore tube with a small-bore tube attached to a larger-bore tube. Tightening in circumferential direction may distribute clamping forces about the line.

The clamp may comprise one or more radial and inward protrusions, which may provide locally increased clamping force and/or provide increased friction, either or both increasing reliability in establishing and/or maintaining a position and/or orientation of the line, e.g. relative to at least the enclosure.

The clamp is adjustable to accommodate and clamp different shapes and/or sizes of lines, e.g. lines of different diameter, e.g. to provide a suitable crimping force around the axis fixing the respective line. The adjustability may be one-directional, e.g. only allowing size reduction, crimping and/or increasing force, or be reversible, e.g. allowing also relaxation and/or size increase.

The clamp may comprise one or more indexing features and/or fixation features for establishing a size and/or a clamping force. In particular, the clamp may comprise indexing features and/or fixation features for defining one or more predetermined relative positions of clamp portions, such as one or more latches and/or ratchets, for establishing different clamp sizes and/or clamping forces. An indexing feature, in particular a latch may facilitate a snap lock for closing the clamp relative to the axis, e.g. encircling the axis.

The clamp may be openable and closable, in an open state accepting a line to be clamped in another direction than axial direction, e.g. sideways in a radial direction, and in a closed state configured for clamping the line. The opening and/or closing of the clamp may be reversible, e.g. facilitating exchanging the line.

The enclosure and/or the clamp may extend over more than 180 degrees around the axis, and preferably fully surrounding the axis.

The enclosure may extend over more than 180 degrees around the axis in an axial position, e.g. in one radial plane and preferably it may fully surround the axis in an axial position, e.g. in one radial plane. This may prevent step edges in axial direction of support structures for the line and/or it may prevent abrupt deviations in forces on the tube or line, which might cause localised pinching, bending and/or damaging of the line.

The retainer is openable and closable, in an open state accepting a line to be fixed in another direction than an axial direction, e.g. sideways, in a radial direction, and in a closed state being configured for retaining and clamping the line by the enclosure and clamp respectively. The opening and/or closing of the retainer may be reversible, e.g. facilitating exchanging the line. In particular, the enclosure may be openable and closable, in an open state accepting a line to be retained in another direction than an axial direction, e.g. sideways in a radial direction, and in a closed state configured for radially retaining the line. The opening and/or closing of the enclosure may be reversible, e.g. facilitating exchanging the line. Also the clamp may be openable and closable, in an open state accepting a line to be clamped in another direction than axial direction, e.g. sideways in a radial direction, and in a closed state configured for clamping the line. The opening and/or closing of the clamp may be reversible, e.g. facilitating exchanging the line.

The enclosure may comprise plural parts hingingly connected for opening and closing the enclosure, e.g. two or more C-shaped parts, the hinging axis may extend in axial direction. Such C-shaped parts may extend over more or less than 180 degrees around the axis, by equal or mutually different amounts. This may provide a large opening and/or otherwise facilitate arranging a line into the closure. The hinge may be arranged offset from the axis. E.g., two or more C-shaped parts may be hingingly connected at adjacent respective tips, enabling moving the parts from a combined C- or O-shape for radially retaining the endotracheal tube, to a more-or-less U- or W-shape for receiving the line. The opening and/or closing of the enclosure may be reversible, e.g. facilitating exchanging the line.

The enclosure may comprise a snap lock for closing. This may facilitate single-handed closing the closure. A snap lock may be particularly effective for closing an enclosure comprising hingingly connected C-shaped parts. A snap lock may be openable, for which a release tab may be provided. Also the clamp may comprise a snap lock for closing.

The clamp may be connected to plural parts of the enclosure and be openable and closeable together with the enclosure. This may facilitate arranging a tube or line into the closure and/or into the clamp.

The clamp may comprise a clamp member, which may be deformable, which is attachable or attached to two enclosure parts that are movable with respect to each other for opening and/or closing the enclosure. Thus the clamp may be deformed, in particular opened (further) or closed (further) in conjunction with deformation, in particular opening(further) or closing (further) of the enclosure.

The clamp is accommodated at least partly within the enclosure. In particular, the clamp may be accommodated in the enclosure in radial and/or in axial direction. The enclosure may cover the clamp in axial and/or radial direction. This may protect at least part of the clamp. Also, this may facilitate operating the closure and the clamp, e.g. one or more of (re-)opening, (re-)closing and (re-)adjusting the clamp, if and where applicable.

The retainer may be movable and adjustable relative to the support member to plural positions on the support member, in particular in at least a lateral direction with respect to a proximal and distal direction. The proximal and distal direction may be associated with the axial direction of the retainer.

The support member may comprise a lateral track and the retainer may be movable, in particular translatable along the track.

The support member and the retainer may comprise cooperating indexing features and/or fixation features for defining and/or fixing one or more relative positions and/or orientations. In particular, the support member and the retainer may comprise one or more latches and/or ratchets. Adjustment of such relative positions and/or orientations may be independent on the retention and/or clamping of the line in the retainer.

The support member may be configured for head-mounting of the fixation device, comprising one or more facial rest portions, e.g. cheek rests, and attachment portions for one or more head bands.

The support member may comprise a bridge for maintaining one or more portions of the support member free from skin contact. The bridge may comprise or support the lateral track.

One or more of the facial rest portions may be provided with a skin contact pad, which may be adhesive and/or detachably attached to the respective facial rest portions, e.g. for replacement. For detachable attachment the support member may comprise cooperating fixation parts, e.g. a clamp.

The fixation device may be provided as an assembly together with a medical line such as a wire, a tube which may be an endotracheal tube, or similar. The assembly may be packaged and/or be sterile.

### BRIEF DESCRIPTION OF THE DRAWINGS

The above-described aspects will hereafter be more explained with further details and benefits with reference to the drawings showing a number of embodiments by way of example.
Fig. 1 shows a fixation device in perspective view;
Fig. 2 is an exploded view of the device of Fig. 1;
Figs. 3-5, 6-8, and 9 show the retainer of the device of Fig. 1 in different perspective views, exploded views, and a partial cross section view, respectively;
Figs. 10-14 show different stages of use of the device;
Fig. 15 shows the device in use on a person's head.

### DETAILED DESCRIPTION OF EMBODIMENTS

It is noted that the drawings are schematic, not necessarily to scale and that details that are not required for understanding the present invention may have been omitted. The terms "upward", "downward", "below", "above", and the like relate to the embodiments as oriented in the drawings, unless otherwise specified. Further, elements that are at least substantially identical or that perform an at least substantially identical function are denoted by the same numeral, where helpful individualised with alphabetic suffixes.

Further, unless otherwise specified, terms like "detachable" and "removably connected" are intended to mean that respective parts may be disconnected essentially without destruction of either part, e.g. excluding structures in which the parts are integral (e.g. welded or molded as one piece), but including structures in which parts are attached by or as mated connectors, fasteners, releasable self-fastening features, etc.

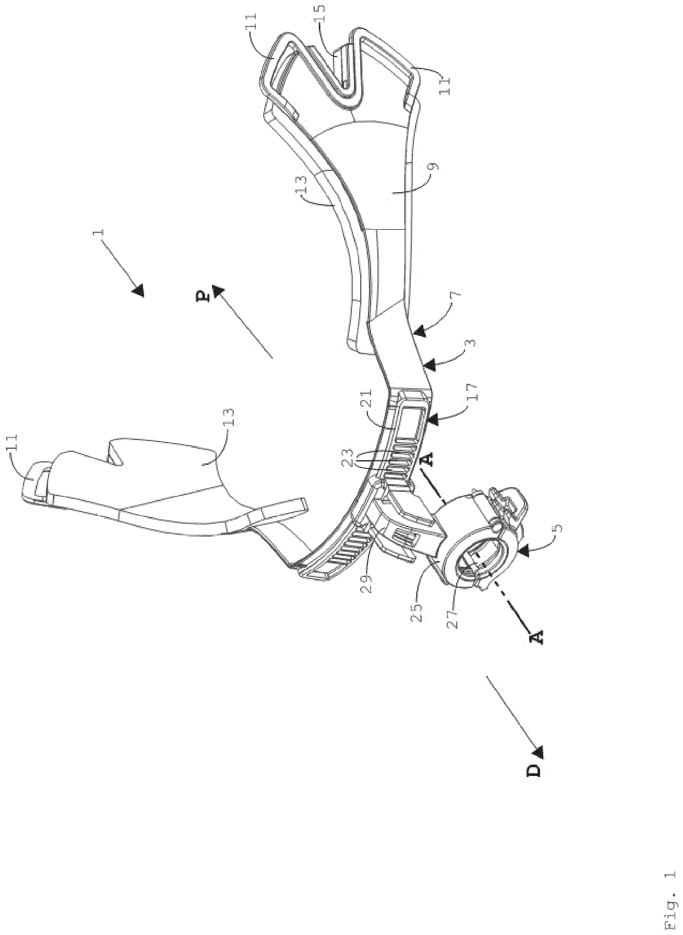

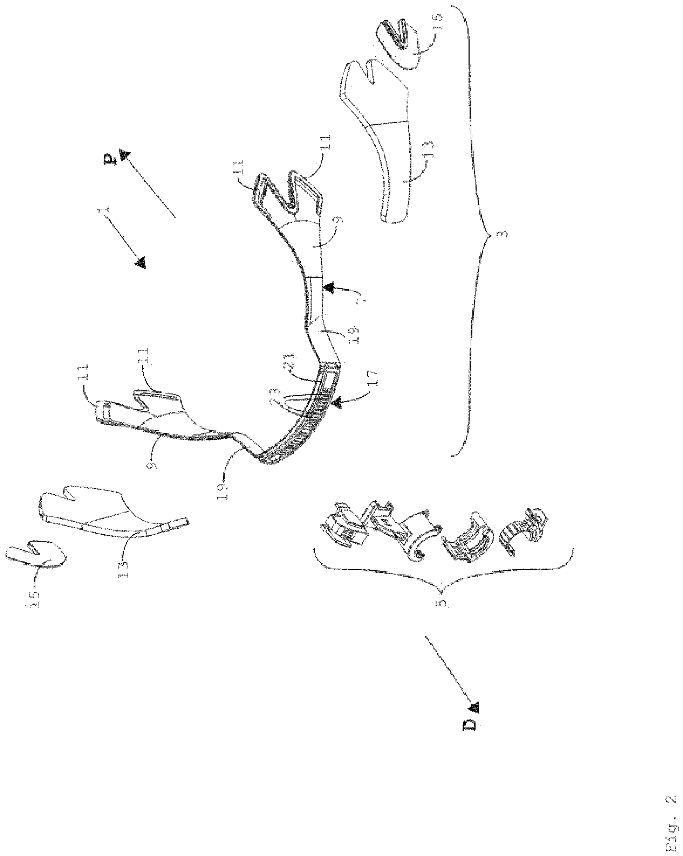

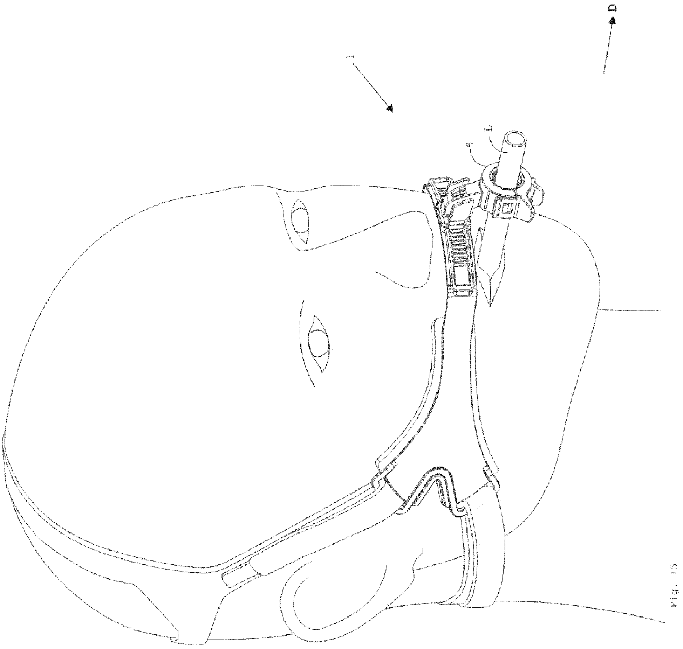

Figs. 1-2 show a fixation device for fixing a medical line to a patient, in particular an endotracheal tube or to the line L as shown in Fig. 15. The fixation device 1 comprises a support member 3 supporting a retainer 5 for retaining the line. The retainer 5 defines an axis A and is adapted to fix the line L extending in axial direction, i.e. over or parallel to the axis A. As best seen in Fig. 15, the support member 3 is configured for mounting the fixation device 1 to the head of a patient , such that the retainer 5 is positioned in front of the mouth or nose of the patient to be intubated and the axis A extends towards the point of intubation (mouth or nose) in proximal - distal relation to the patient, as indicated with arrows P and D, respectively.

The shown support member 3 comprises a main member 7 which comprises facial rest portions, here cheek rests 9, and attachment portions 11 for one or more head bands, e.g. as shown in Fig. 15. The head bands may be elastic or non-elastic and they may be size adjustable, e.g. by doubling up of a band portion to form a loop and attachment of one end to an adjacent portion in a desired position using one or more displaceable buckles, a hook-and-loop type fastener (like Velcro^{®} or similar) as indicated in Fig. 15, or other attachment technique like buttons, zippers, ratchet-portions (like tie-rips or similar). The shown cheek rests 9 are provided with cushioning skin contact pads 13, removably attached to the main member 7 with clamps 15 that are detachably fixable to the main member 7. The pads 13 may be adhesive but preferably they are non-adhesive. The materials of the contact pads 13 and clamps 15 may differ. The pads 13 may be flexible, or at least comprise a flexible material, e.g. a foam. The pads may comprise a coating, a laminate and/or another surface finish, e.g. a density gradient in a foam-material. The clamps 15 are generally solid, preferably resilient. The contact pads 13 and clamps 15 may be attached, preferably fixed, together from separate parts, e.g. by stitching, gluing, welding, etc., or they may be formed as a unitary object. A surface finish on one or both of a pad 13 and a clamp 15 may facilitate attachment of them together. The attachment may be substantially permanent or detachable.

Non-adhesive and/or exchangeable pads 13 may reduce or prevent skin damage, in particular in case of prolonged use. Also, different skin pad sizes and/or textures may be used. The main member 7 here is generally formed as a bridge, supporting a track 17 on the cheek rests 9 by struts 19. thus the track 17 may be kept separated from the face of the patient, in particular from the patient's lips and nose (cf. Fig. 15).

The track 17 extends lateral to the axis A (Fig. 1) and comprises rails 21 and a series of teeth 23.

In other embodiments, not shown, the retainer 5 may be supported on a differently formed support member for mounting on another patient body part, e.g. for fixing a catheter, a drain, a sensor line and/or an infusion line.

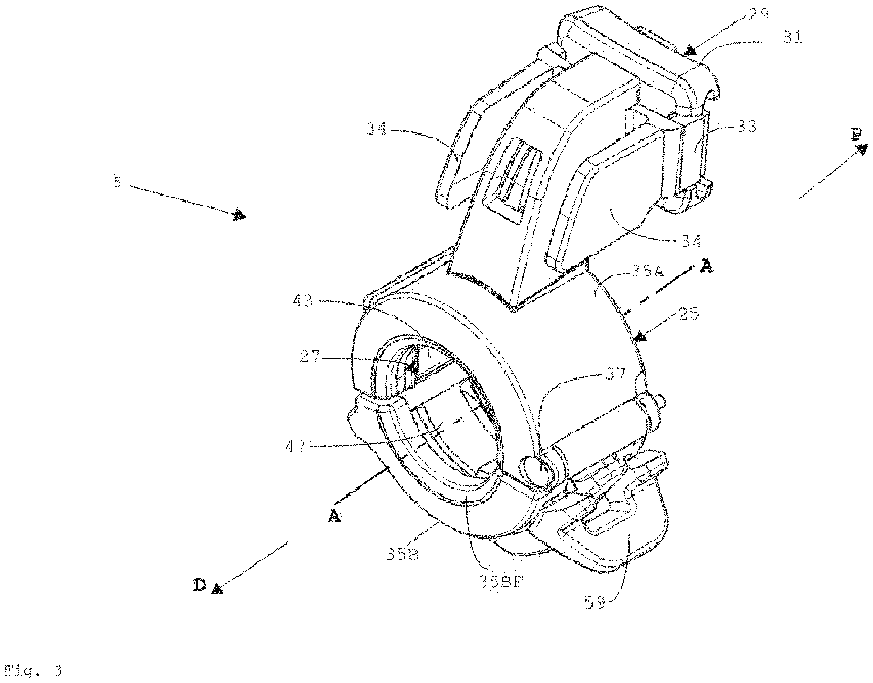

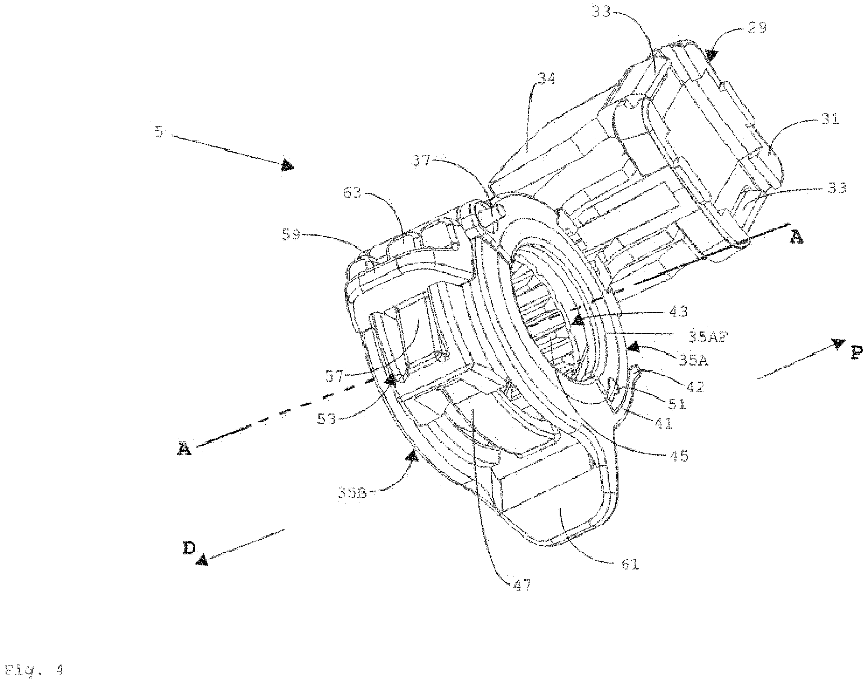

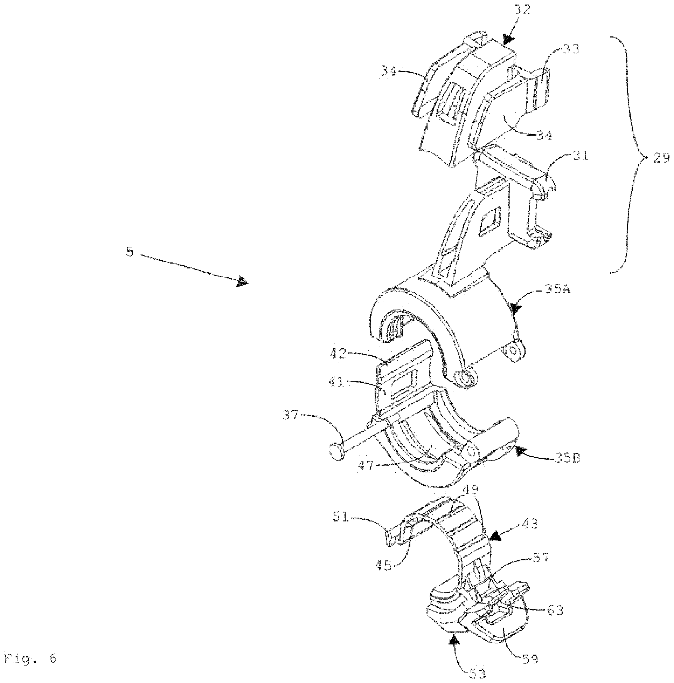

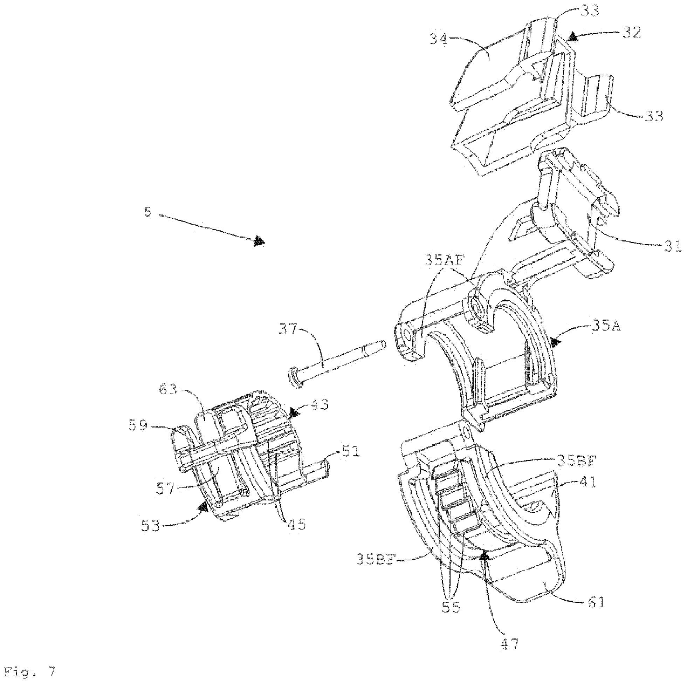

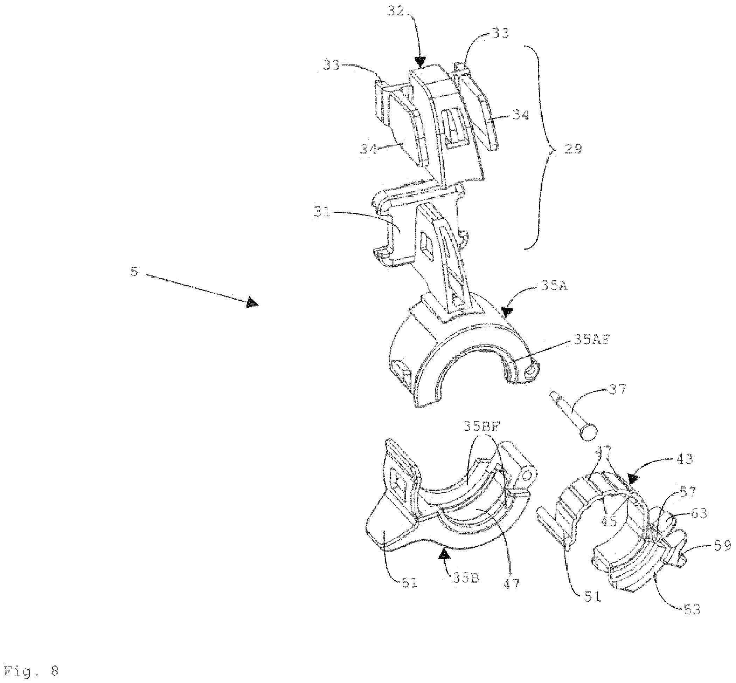

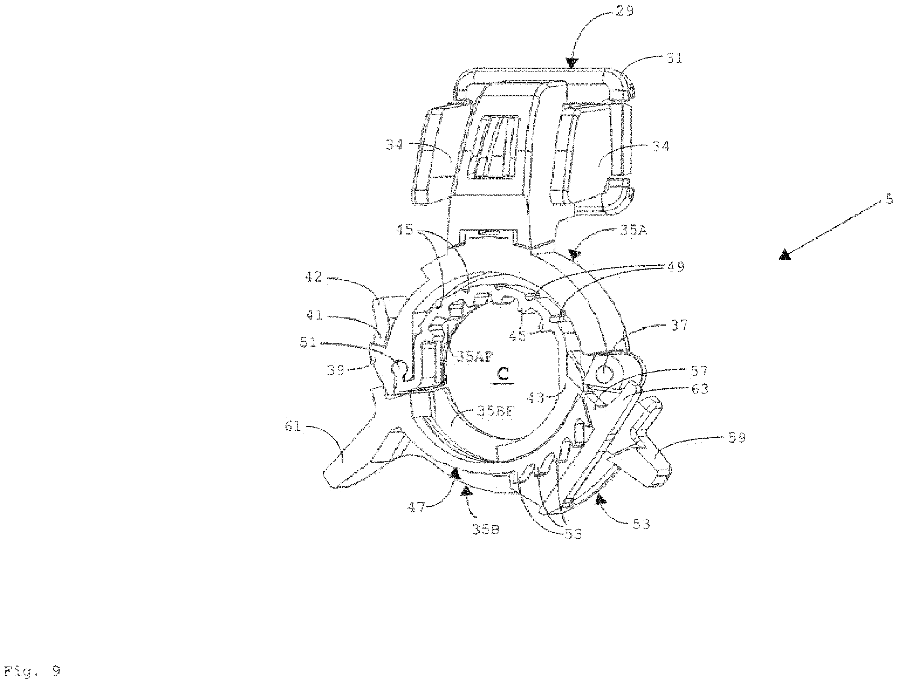

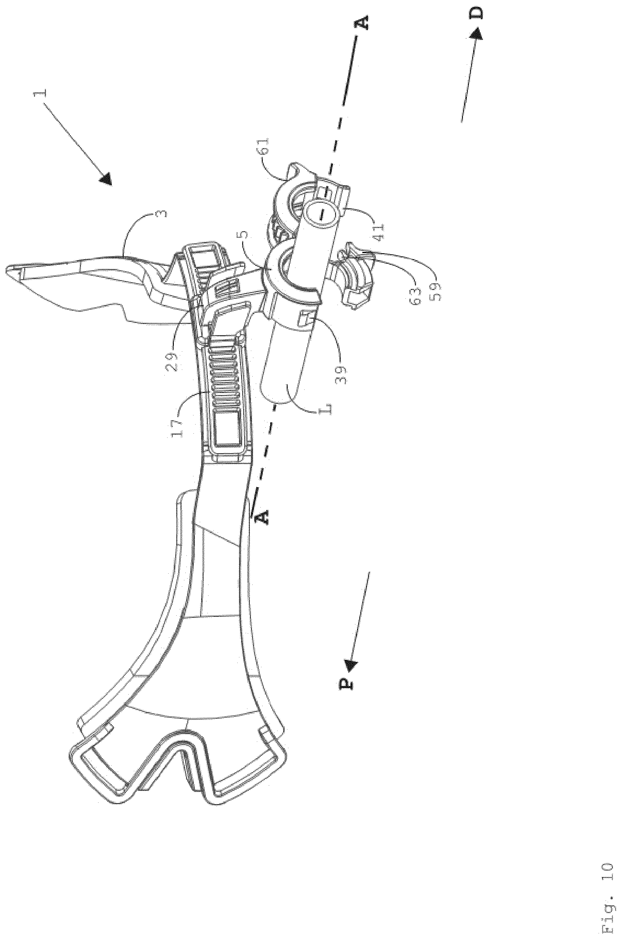

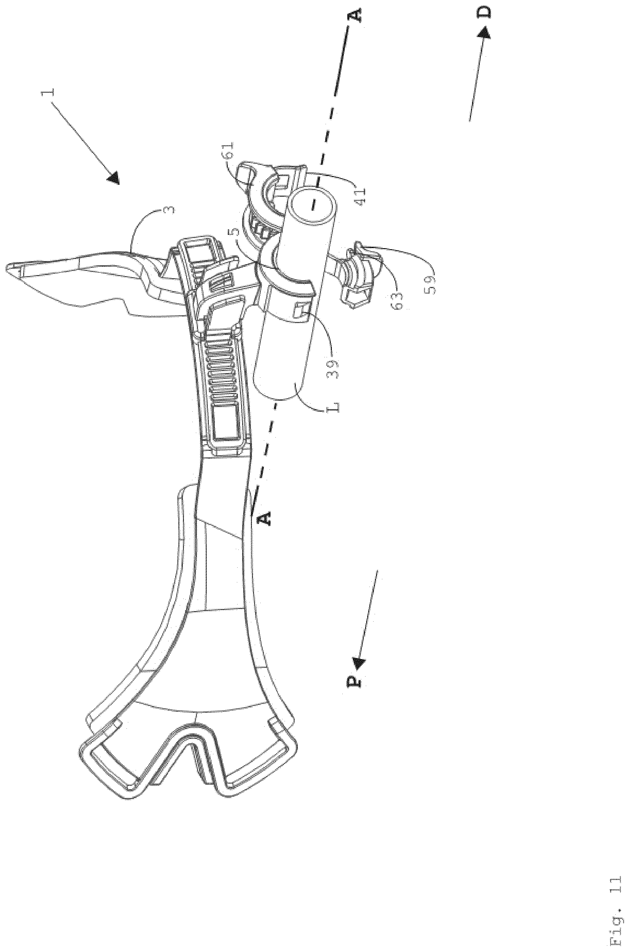

Figs. 3-8 are various views of the retainer 5 separately. Fig. 9 is a cross section view. Figs. 10-13 show use of the device (partially shown). Referring to Figs. 3-9, the retainer 5 comprises an enclosure 25 for radially retaining the line in axial direction along the axis A, a clamp 27, and further a mount 29 for connecting the retainer 5 to the support member 3, in particular to the track 17 thereof.

In the shown embodiment the mount 29 comprises a connector 31 for slidably coupling the retainer 5 to the rails 21 of the track 17 and pawls 33 for engaging teeth 23 of the track 17. Here, the pawls 33 are provided on a separate cap 32 fitted to the connector 31, e.g. by snapping. Combining separate retainer parts (e.g. here connector 31 and cap 32) facilitates selecting different materials for the different retainer parts and/or simplifying a mold in case one or more parts of the retainer 5 are molded. The pawls 33 are movable relative to the connector 31 using tabs 34. Thus, when (the mount 29 of) the retainer 5 is operably coupled to the track 17 (see Fig. 1) the pawls 33 can be selectively made to engage/disengage teeth 23 of the track 17 so that the retainer 5 can be slid laterally along the track 17 or be fixed to the latter in several desired relative positions.

The shown enclosure 25 comprises two arms 35A, 35B formed as C-shaped parts hingingly connected to each other at respective tips, here with a pivot 37 which extends parallel to the axis A and offset from the latter, but other hinges, e.g. one or more film hinges, may be provided.

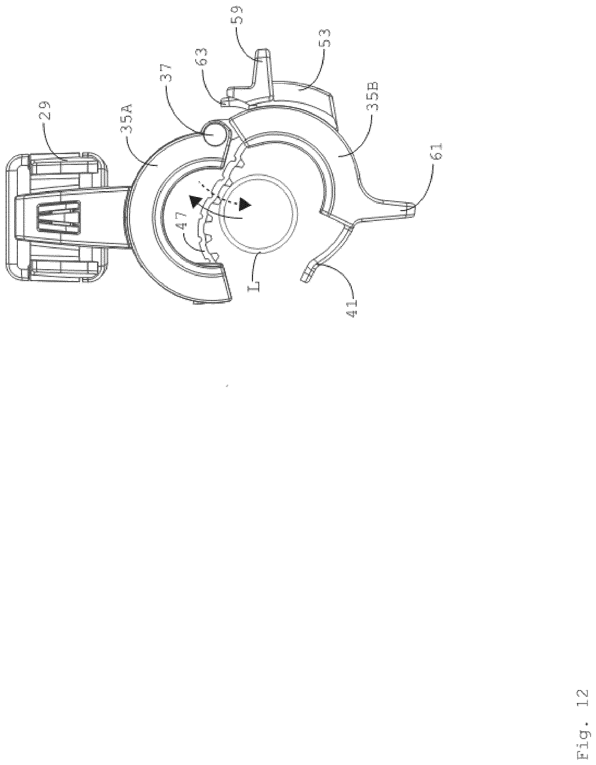

Due to the hinging connection of the arms 35A, 35B, the arms 35A, 35B are movable from a closed configuration, here a combined O-shape (Figs 3-5, 9) for radially retaining the line (Fig. 12), to an open configuration for receiving the line (Figs. 10-11) in which open configuration, the arms 35A, 35B together may form a more-or-less U- or W-shape, depending on the amount of opening and the relative positions of the arms 35A, 35B. Here, the arms 35A, 35B each are substantially semicircular but other shapes such as elliptical or rectangular may be used.

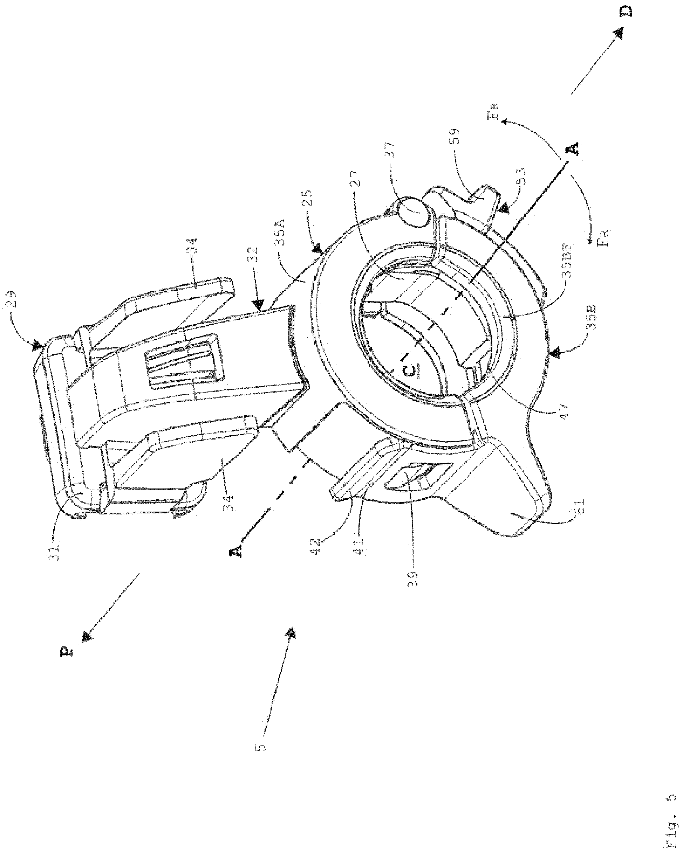

The enclosure 25 comprises a latch 39 on one arm 35A and a matching catch 41 on the other arm 35B which together form a snap lock for closing the enclosure 25 such that it forms a fully closed device encircling the axis A and providing an open channel C. Note that each arm 35A, 35B comprises, on axially opposite ends, radial inward extending flange portions 35AF, 35BF which are located in a common radial plane and provide rings around the axis A. The flange portions 35AF, 35BF may support the line on opposite sides of the clamp 27 (see below). Such opposite support, in particular if extending over more than 180 degrees around the axis as here, can absorb lateral forces, e.g. rotation forces F_{R} if the line and/or the device is pulled sideways (see Fig. 5), e.g. by movement of a patient's head so that the clamp 27 may be, at least largely, spared from such forces F_{R}, relaxing robustness requirements for the clamp 27.

Provision of an optional release to the lock (39 + 41), here in the form of a tab 42, facilitates opening of the enclosure 25, such that that can be reversibly, and preferably repeatedly, closed and opened. Note that other locks than a snap lock may be used.

The clamp 27 comprises a clamp member 43 which comprises radial inward protrusions 45. The clamp 27 further comprises a second clamp member 47.

The shown clamp member 43 is flexibly deformable, preferably resilient, which properties may be due to material choices and/or shaping of the clamp member 43, e.g. by recesses 49 reducing thickness variations by the protrusions 45.

In the shown embodiment, the clamp member 43 is attached on one side to one arm 35A, here being attached with a key 51 fit in a matching groove in the arm 35A and extending parallel to the axis A and offset from the latter. The clamp member 43 is flexible allowing movement and deformation with respect to the key 51 and the arm 35A. In another embodiment (not shown), instead of a key and groove connection, hinges, e.g. including a pivot and/or including one or more film hinges, may be provided. Also or alternatively, the clamp member may be integral with the enclosure (not shown). An opposite side of the clamp member 43 is attached to a clamp connector 53 with is in turn attached to the second clamp member 47. The clamp member 43 extends, as shown, over more than 180 degrees around the axis A.

The second clamp member 47 is attached on one side to one arm 35B. Here, as an option, the second clamp member 47 is formed integral with the arm 35B so that the second clamp member 47 may be seen as a portion of the arm 35B. However, the second clamp member 47 may also be a separate part hingingly attached with a pivot or other hinge to the enclosure, like the clamp member 43 discussed above.

The second clamp member 47 may be, as intended here, be resiliently flexible. The clamp member 43 and the second clamp member 47 are adjustably couplable or coupled together via the clamp connector 53. Thus coupled, the clamp 27 is openable, here on an opposite side, by opening of the enclosure 25 so that a line to be clamped can be introduced into the clamp 27 from the open side; see Figs. 10-11. In closed configuration of the enclosure 25 the (e.g. see Figs. 1, 3-5, 9) the clamp 27 extends fully around the axis A.

Visible in Fig. 12, is that since the clamp member 43 and the second clamp member 47 are attached to different arms 35A, 35B of the enclosure 25, in an open configuration of the retainer 5, the clamp member 43 and the second clamp member 47 are deformed. Introducing a line L into the retainer 5 may cause that the line L engages the clamp member 43 and, on pressing the line into the retainer 5 further (see solid arrow in Fig. 12), urges the clamp member 43 (more) into the arm 35A, thus pulling both arms 35A, 35B towards a closed configuration. Thus, stretching or opening of the clamp 27 and causing deformation of the retainer 5 towards a holding and/or clamping configuration by insertion of the line L to be clamped itself, ease of use is increased and proper alignment of the line L to a position for retainment and clamping is assisted. Note that, in reverse, on opening of the retainer 5 holding a clamped line L, stretching the clamp member 43 assists ejecting and removing the line L from the retainer 5, as indicated in Fig. 12 with a dashed arrow.

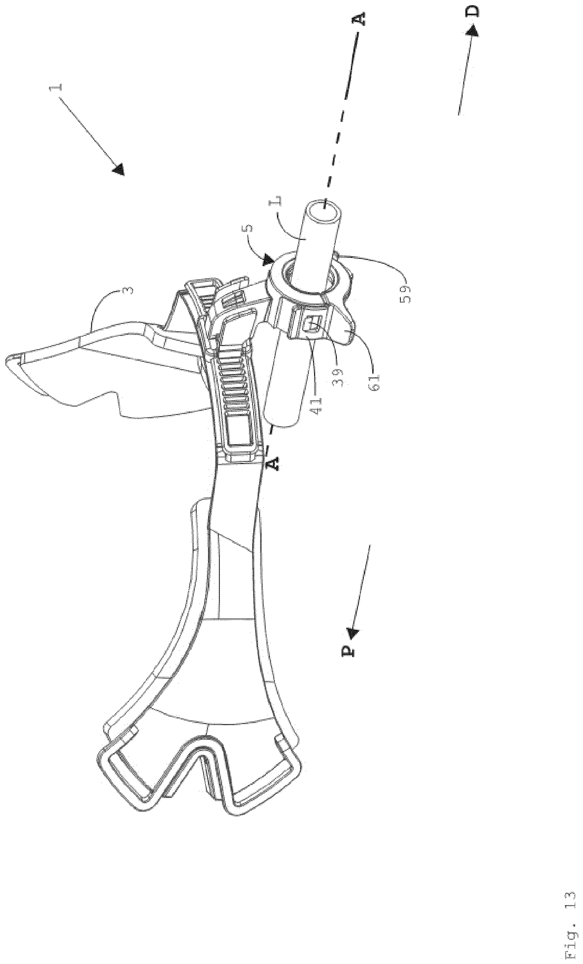

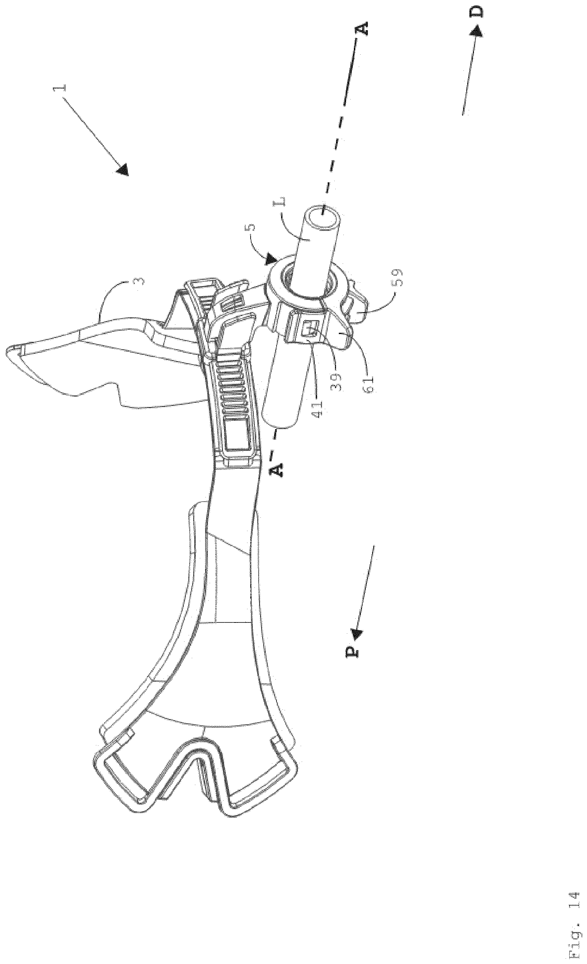

The connector 53 is movable over the second clamp member 47. The second clamp member 47 and the clamp connector 53 comprise, respectively teeth 55 and a pawl 57 forming a ratchet-and-pawl connection. By moving the clamp connector 53 and the second clamp member 47 relative to each other, in particular in closed configuration of (the enclosure 27 of) the retainer 5 and therewith of the clamp 27, the clamp 27 is tightenable in circumferential direction around the axis A so that an opening of the clamp 27 accommodating the line is reduced. This is comparable to tightening a cable binder of the so-called "tie wrap"- or "tie rip"-types. Thus a crimping force can be provided on the line L. For adjusting the relative position of the connector to the second clamp member 47 the connector 53 is optionally provided with a tab 59 and (an arm 35B of) the enclosure 25 is optionally provided with a tab 61; the tabs 59, 61 may be pulled together by pinching, which may e.g. be done by a thumb and forefinger of one hand, see also Figs. 13 and 14.

For releasing the clamp 27 once it is tightened, the retainer 5 may be opened as a whole (here, by unlocking the latch 39 and catch 41 snap lock), or the pawl 57 may be withdrawn to disengage it from the teeth 55. For such release of the clamp 27 the pawl 57 may be provided with a tab 63.

The present retainer 5 can accommodate lines L of any diameter fitting inside the enclosure 25 when that is closed, and it can clamp lines of various smaller diameter than the maximum diameter, depending on, i.a., flexibility of the clamp members 43, 47 and relative sizes of the clamp connector 53.

The disclosure is not restricted to the above described embodiments which can be varied in a number of ways within the scope of the claims. For instance, the support member may have different shapes. The retainer may be larger or smaller. Plural retainers may be connected to one support member. Plural retainers for different lines may be connected to each other and be connected to a single support member as an assembly. The retainer may be fixed to a support member. The support member may not have a bridge portion but be configured for fully contacting a patient. Tabs may be made smaller or larger. The retainer may have a longer or shorter size in axial direction.

The head band may have another shape and/or be attached differently.

The device may be combined with a bite-block, known per se. The latter may be coupled to the support member and/or the retainer 5, e.g. by a coupling to the mount 29. The latter may e.g. be facilitated by adaptation of the cap 32.

## Claims

1. A fixation device (1) for fixing a medical line (L) to a patient, in particular an endotracheal tube, comprising
a retainer (5) and a support member (3) for supporting the retainer (5) on the patient, the retainer (5) defining an axis (A) and being adapted to retain the line (L) extending in axial direction,
wherein the retainer (5) is openable and closable, in an open state accepting the line (L) to be clamped in another direction than axial direction, and in a closed state configured for clamping the line;
wherein the retainer (5) comprises
an enclosure (25) for radially retaining the line (L) and a clamp (27) accommodated at least partly within the enclosure (25),wherein the clamp (27) comprises a deformable clamp member (43),
**characterised in that**
the deformable clamp member (43) comprises one of a flexible strap, belt or chain, that the clamp (27) comprises a second clamp member (47)which is adjustably coupled together with the deformable clamp member (43)via a clamp connector (53), wherein the second clamp member (47) and the clamp connector (53) comprise, respectively, teeth (55) and a pawl (57) forming a ratchet-and-pawl connection for tightening the clamp (27) in the circumferential direction around the axis (A), so that an opening of the clamp (27) accommodating the line (L) is reduced and the clamp (27) is configured to provide a crimping force around the axis (A) for fixing the line (L) in at least axial direction relative to the enclosure (25), preferably also in radial direction.

2. The fixation device (1) of claim 1, wherein the clamp (27) is configured to provide a substantially axisymmetric clamping force distribution.

3. The fixation device (1) of any preceding claim, wherein the clamp (27) is adjustable to accommodate and clamp different shapes and/or sizes of lines (L).

4. The fixation device (1) of any preceding claim, wherein the clamp (27) comprises one or more indexing features (55) and/or fixation features (57) for establishing a size and/or a clamping force.

5. The fixation device (1) of any preceding claim, wherein the retainer (5) is in an open state accepting a line (L) to be clamped sideways in a radial direction.

6. The fixation device (1) of any preceding claim, wherein the enclosure (25) and/or the clamp (27) extends over more than 180 degrees around the axis (A) in an axial position, e.g. in one radial plane, preferably fully surrounding the axis (A) in the axial position.

7. The fixation device (1) of any preceding claim, wherein the enclosure (25) comprises plural parts (35A, 35B) hingingly connected for opening and closing the enclosure (25), in particular two or more C-shaped parts (35A, 35B) hingingly connected at adjacent respective tip ends.

8. The fixation device (1) of any preceding claim, wherein at least one of the enclosure (25) and the clamp comprises (27) a snap lock (39, 41) for closing.

9. The fixation device (1) of any preceding claim,
wherein the clamp (27) is connected to plural parts (35A, 35B) of the enclosure (25) and is (re-)openable and/or (re-)closeable together with the enclosure (25).

10. The fixation device (1) of any preceding claim,
wherein the deformable clamp member (43)is attachable or attached to two enclosure parts (35A, 35B) that are movable with respect to each other for opening and/or closing the enclosure (25) .

11. The fixation device (1) of any preceding claim,
wherein the retainer (5) is movable and adjustable relative to the support member (3) to plural positions on the support member (3), in particular movable and adjustable in at least a lateral direction with respect to a proximal and distal direction (P; D).

12. The fixation device (1) of any preceding claim,
wherein the support member (3) comprises a lateral track (17) and the retainer (5) is movable along the track (17), the support member (3) and the retainer (5) possibly comprising cooperating indexing features (23) and/or fixation features (33) for defining and/or fixing one or more relative positions and/or orientations.

13. The fixation device (1) of any preceding claim,
wherein the support member (3) is configured for head-mounting of the fixation device (1), comprising one or more facial rest portions, e.g. cheek rests (9), and attachment portions (11) for one or more head bands, and/or
wherein the support member comprises a bridge for maintaining one or more portions of the support member free from skin contact, which bridge may comprise or support the lateral track (17) as specified in claim 12.

## Patentansprüche

1. Fixierungsvorrichtung (1) zum Fixieren einer medizinischen Leitung (L) an einem Patienten, insbesondere eines Endotrachealtubus, Folgendes umfassend:
eine Halterung (5) und ein Stützelement (3) zum Abstützen der Halterung (5) am Patienten, wobei die Halterung (5) eine Achse (A) definiert und dazu eingerichtet ist, die sich in Axialrichtung erstreckende Leitung (L) zu halten,
wobei die Halterung (5) geöffnet und geschlossen werden kann, im geöffneten Zustand die in einer anderen Richtung als der Axialrichtung einzuspannende Leitung (L) aufnimmt und im geschlossenen Zustand dazu ausgelegt ist, die Leitung einzuspannen;
wobei die Halterung (5) Folgendes umfasst:
ein Gehäuse (25) zum radialen Halten der Leitung (L) und eine Klammer (27), die zumindest teilweise in dem Gehäuse (25) untergebracht ist, wobei die Klammer (27) ein verformbares Klammerelement (43) umfasst,
**dadurch gekennzeichnet, dass**
das verformbare Klammerelement (43) ein flexibles Band, einen flexiblen Gurt oder eine flexible Kette umfasst, dass die Klammer (27) ein zweites Klammerelement (47) umfasst, das über einen Klammerverbinder (53) mit dem verformbaren Klammerelement (43) verstellbar zusammengekoppelt ist, wobei das zweite Klammerelement (47) und der Klammerverbinder (53) Zähne (55) bzw. eine Sperrklinke (57) umfassen, die eine Sperrklinkenverbindung zum Anziehen der Klammer (27) in Umfangsrichtung um die Achse (A) ausbilden, sodass eine Öffnung der Klammer (27), die die Leitung (L) aufnimmt, verkleinert wird und die Klammer (27) dazu ausgelegt ist, zum Fixieren der Leitung (L) in mindestens der Axialrichtung in Bezug zum Gehäuse (25), vorzugsweise auch in Radialrichtung, eine Quetschkraft um die Achse (A) vorzusehen.

2. Fixierungsvorrichtung (1) nach Anspruch 1, wobei die Klammer (27) dazu ausgelegt ist, eine im Wesentlichen achsensymmetrische Einspannkraftverteilung vorzusehen.

3. Fixierungsvorrichtung (1) nach einem der vorstehenden Ansprüche, wobei die Klammer (27) einstellbar ist, um verschiedene Formen und/oder Größen der Leitungen (L) aufzunehmen und einzuspannen.

4. Fixierungsvorrichtung (1) nach einem der vorstehenden Ansprüche, wobei die Klammer (27) einen oder mehrere Schaltmerkmale (55) und/oder Fixierungsmerkmale (57) zum Einrichten einer Größe und/oder einer Einspannkraft umfasst.

5. Fixierungsvorrichtung (1) nach einem der vorstehenden Ansprüche, wobei sich die Halterung (5) in einem geöffneten Zustand befindet, wenn sie eine Leitung (L) aufnimmt, die in eine Radialrichtung seitlich einzuspannen ist.

6. Fixierungsvorrichtung (1) nach einem der vorstehenden Ansprüche, wobei sich das Gehäuse (25) und/oder die Klammer (27) in einer Axialstellung über mehr als 180 Grad um die Achse (A) erstreckt, z. B. in einer Radialebene, und vorzugsweise die Achse (A) in der Axialstellung vollständig umgibt.

7. Fixierungsvorrichtung (1) nach einem der vorstehenden Ansprüche, wobei das Gehäuse (25) mehrere Teile (35A, 35B) umfasst, die gelenkig verbunden sind, um das Gehäuse (25) zu öffnen und zu schließen, insbesondere zwei oder mehrere C-förmige Teile (35A, 35B), die an jeweils angrenzenden Spitzenenden gelenkig verbunden sind.

8. Fixierungsvorrichtung (1) nach einem der vorstehenden Ansprüche, wobei das Gehäuse (25) und/oder die Klammer (27) einen Schnappverschluss (39, 41) zum Verschließen umfasst.

9. Fixierungsvorrichtung (1) nach einem der vorstehenden Ansprüche, wobei die Klammer (27) mit mehreren Teilen (35A, 35B) des Gehäuses (25) verbunden ist und zusammen mit dem Gehäuse (25) (erneut) geöffnet und/oder (erneut) geschlossen werden kann.

10. Fixierungsvorrichtung (1) nach einem der vorstehenden Ansprüche, wobei das verformbare Klammerelement (43) an zwei Gehäuseteilen (35A, 35B) befestigbar oder befestigt ist, die in Bezug zueinander beweglich sind, um das Gehäuse (25) zu öffnen und/oder zu schließen.

11. Fixierungsvorrichtung (1) nach einem der vorstehenden Ansprüche,
wobei die Halterung (5) in Bezug zum Stützelement (3) in mehrere Stellungen am Stützelement (3) beweglich und einstellbar ist, insbesondere in mindestens eine laterale Richtung in Bezug zu einer proximalen und distalen Richtung (P; D) beweglich und einstellbar ist.

12. Fixierungsvorrichtung (1) nach einem der vorstehenden Ansprüche, wobei das Stützelement (3) eine laterale Schiene (17) umfasst und die Halterung (5) entlang der Schiene (17) beweglich ist, wobei das Stützelement (3) und die Halterung (5) möglicherweise zusammenwirkende Schaltmerkmale (23) und/oder Fixierungsmerkmale (33) zum Definieren und/oder Fixieren einer oder mehrerer relativer Stellungen und/oder Ausrichtungen umfassen.

13. Fixierungsvorrichtung (1) nach einem der vorstehenden Ansprüche, wobei das Stützelement (3) zur Kopfmontage der Fixierungsvorrichtung (1) ausgelegt ist, umfassend eine oder mehrere Gesichtsablageabschnitte, z. B. Wangenablagen (9), und Befestigungsabschnitte (11) für ein oder mehrere Kopfbänder, und/oder wobei das Stützelement eine Brücke umfasst, um den Hautkontakt eines oder mehrerer Abschnitte des Stützelements zu verhindern, wobei die Brücke die laterale Schiene (17) nach Anspruch 12 umfassen oder abstützen kann.

## Revendications

1. Dispositif de fixation (1) pour la fixation d'un tube médical (L) à un patient, en particulier un tube endotrachéal, comprenant
un dispositif de retenue (5) et un élément de support (3) pour supporter le dispositif de retenue (5) sur le patient, le dispositif de retenue (5) définissant un axe (A) et étant propre à retenir le tube (L) s'étendant dans une direction axiale,
le dispositif de retenue (5) pouvant être ouvert et fermé, celui-ci recevant, dans l'état ouvert, le tube (L) à assujettir dans une direction autre que la direction axiale et étant conçu pour assujettir le tube dans l'état fermé ;
le dispositif de retenue (5) comprenant
une enveloppe (25) destinée à retenir radialement le tube (L) et un dispositif d'assujettissement (27) logé au moins partiellement à l'intérieur de l'enveloppe (25), le dispositif d'assujettissement (27) comprenant un élément d'assujettissement déformable (43),
**caractérisé en ce que**
l'élément d'assujettissement déformable (43) comprend l'une d'une sangle flexible, une courroie et une chaîne, **en ce que** le dispositif d'assujettissement (27) comprend un second élément d'assujettissement (47) qui est accouplé de manière réglable à l'élément d'assujettissement déformable (43) par le biais d'un système de raccordement de dispositif d'assujettissement (53), le second élément d'assujettissement (47) et le système de raccordement de dispositif d'assujettissement (53) comprenant, respectivement, des dents (55) et un cliquet (57) formant un mécanisme de raccordement à rochet et cliquet pour serrer le dispositif d'assujettissement (27) dans la direction circonférentielle autour de l'axe (A), de telle sorte qu'une ouverture du dispositif d'assujettissement (27) recevant le tube (L) soit réduite et le dispositif d'assujettissement (27) étant conçu pour produire une force de compression autour de l'axe (A) pour fixer le tube (L) au moins dans la direction axiale relativement à l'enveloppe (25), de préférence également dans la direction radiale.

2. Dispositif de fixation (1) selon la revendication 1, dans lequel le dispositif d'assujettissement (27) est conçu pour produire une distribution de force d'assujettissement sensiblement axisymétrique.

3. Dispositif de fixation (1) selon l'une quelconque des revendications précédentes, dans lequel le dispositif d'assujettissement (27) est réglable pour recevoir et assujettir des tubes (L) de différentes formes et/ou tailles.

4. Dispositif de fixation (1) selon l'une quelconque des revendications précédentes, dans lequel le dispositif d'assujettissement (27) comprend un ou plusieurs éléments de positionnement (55) et/ou éléments de fixation (57) pour établir une taille et/ou une force d'assujettissement.

5. Dispositif de fixation (1) selon l'une quelconque des revendications précédentes, dans lequel le dispositif de retenue (5), dans l'état ouvert, reçoit un tube (L) à assujettir latéralement dans une direction radiale.

6. Dispositif de fixation (1) selon l'une quelconque des revendications précédentes, dans lequel l'enveloppe (25) et/ou le dispositif d'assujettissement (27) s'étendent sur plus de 180 degrés autour de l'axe (A) dans une position axiale, par ex. dans un plan radial, entourant de préférence entièrement l'axe (A) dans la position axiale.

7. Dispositif de fixation (1) selon l'une quelconque des revendications précédentes, dans lequel l'enveloppe (25) comprend plusieurs parties (35A, 35B) raccordées de manière articulée pour ouvrir et fermer l'enveloppe (25), en particulier au moins deux parties en C (35A, 35B) raccordées de manière articulée au niveau d'extrémités terminales respectives adjacentes.

8. Dispositif de fixation (1) selon l'une quelconque des revendications précédentes, dans lequel l'enveloppe (25) et/ou le dispositif d'assujettissement (27) comprennent un système de blocage à emboîtement-pression (39, 41) pour la fermeture.

9. Dispositif de fixation (1) selon l'une quelconque des revendications précédentes, dans lequel le dispositif d'assujettissement (27) est raccordé à plusieurs parties (35A, 35B) de l'enveloppe (25) et peut être (r)ouvert et/ou (re)fermé conjointement avec l'enveloppe (25).

10. Dispositif de fixation (1) selon l'une quelconque des revendications précédentes, dans lequel l'élément d'assujettissement déformable (43) peut être ou est attaché à deux parties d'enveloppe (35A, 35B) qui sont mobiles l'une par rapport à l'autre pour ouvrir et/ou fermer l'enveloppe (25).

11. Dispositif de fixation (1) selon l'une quelconque des revendications précédentes, dans lequel le dispositif de retenue (5) est mobile et réglable par rapport à l'élément de support (3) de façon à pouvoir le placer dans plusieurs positions sur l'élément de support (3), en particulier est mobile et réglable au moins dans une direction latérale par rapport à une direction proximale/distale (P ; D).

12. Dispositif de fixation (1) selon l'une quelconque des revendications précédentes, dans lequel l'élément de support (3) comprend une glissière latérale (17) et le dispositif de retenue (5) est déplaçable le long de la glissière (17), l'élément de support (3) et le dispositif de retenue (5) comprenant éventuellement des éléments de positionnement (23) et/ou des éléments de fixation (33) coopérants pour définir et/ou fixer une ou plusieurs positions et/ou orientations relatives.

13. Dispositif de fixation (1) selon l'une quelconque des revendications précédentes, dans lequel l'élément de support (3) est conçu en vue d'une installation sur la tête du dispositif de fixation (1), et comprend à cette fin une ou plusieurs parties d'appui facial, par ex. des appuis de joues (9), et parties d'attache (11) pour un ou plusieurs bandeaux, et/ou
dans lequel l'élément de support comprend un pont pour maintenir une ou plusieurs parties de l'élément de support à distance de la peau, ledit pont pouvant comprendre ou supporter la glissière latérale (17) décrite dans la revendication 12.
